# EUROPEAN PATENT SPECIFICATION

(11) **EP 1 893 161 B1**
(45) Date of publication and mention of the grant of the patent: **26.08.2015**
(21) Application number: 06777318.4
(22) Date of filing: 14.06.2006
(51) Int. Cl.: A61K 8/34, A61K 8/37, A61K 8/42, A61K 8/44, A61K 31/196, A61Q 9/02, A61Q 15/00, A61Q 19/00

(54) **MIXTURES COMPRISING ANTHRANILIC ACID AMIDES AND COOLING AGENTS AS COSMETIC AND PHARMACEUTICAL COMPOSITIONS FOR ALLEVIATING ITCHING**
GEMISCHE MIT ANTHRANILINSÄUREAMIDEN UND KÜHLENDEN MITTELN ALS KOSMETISCHE UND PHARMAZEUTISCHE ZUSAMMENSETZUNGEN ZUR LINDERUNG VON JUCKREIZ
MELANGES COMPRENANT DES AMIDES DE L'ACIDE ANTHRANILIQUE ET DES AGENTS DE REFROIDISSEMENT, EN TANT QUE COMPOSITIONS COSMETIQUES ET PHARMACEUTIQUES POUR ATTENUER LES DEMANGEAISONS

(30) Priority: 14.06.2005 US 690153 P; 23.05.2006 EP 06230506
(43) Date of publication of application: 05.03.2008
(73) Proprietor: Symrise AG, 37603 Holzminden (DE)
(72) Inventor: VIELHABER, Gabriele, 37603 Holzminden (DE); SCHMAUS, Gerhard, 37671 Höxter-Bosseborn (DE); PILLAI, Ravikumar, Emerson, New Jersey 07630 (US)
(74) Representative: Fabry, Bernd
(86) International application number: PCT/EP2006/063175
(87) International publication number: WO 2006/134120

(56) References cited:
- EP-A- 0 993 827
- EP-A- 1 430 880
- WO-A-2004/047833
- US-A- 4 070 484
- US-A- 5 266 592

## Description

The present invention relates to mixtures of anthranilic acid amide and cooling agents which can be used especially as cosmetic and pharmaceutical compositions for alleviating itching and/or the reduction of skin reddening.

WO 2004/047833, on which the present invention is based, discloses that certain anthranilic acid amides (of Formula 1) inhibit the substance P-induced release of histamines from mast cells and thus are suitable as cosmetic and pharmaceutical compositions for alleviating itching. The compounds of Formula 1 indicated in WO 2004/047833 are also particularly preferred for use within the framework of the present invention.

WO 2004/047833 discloses that the use concentration of a particular compound of Formula 1 is up to 10 percent by mass, based on the total weight of a ready-to-use cosmetic or pharmaceutical end product. In some cases, however, such high use concentrations seem to be problematic for cosmetic and/or pharmaceutical reasons relating e.g. to formulation technology.

The object of the present invention was therefore to provide mixtures active in the alleviation of itching and/or the reduction of skin reddening which contain, in addition to the compound of Formula 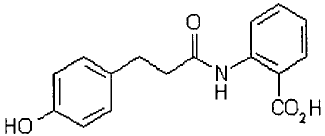 another compound which interacts synergistically with the compound of the Formula so that even low use concentration of the compound of the Formula is sufficient to produce a good effect in the alleviation of itching or the reduction of reddening.

This object is achieved according to the invention by the provision of a mixture comprising or consisting of:
(a) compound of Formula 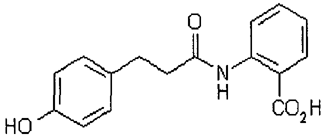 also representing the corresponding cosmetically or pharmaceutically acceptable salts and solvates;
   and
(b) one or more cooling agents selected from the group consisting of I-menthol, menthone glycerol acetal and menthyl lactate (preferably I-menthyl lactate, especially I-menthyl I-lactate).

Although it is already known from WO 2004/047833 that a cosmetic preparation can contain, in addition to a compound of Formula, other active compounds for alleviating reddening and itching, WO 2004/047833 does not disclose that the addition of cooling agents leads to a synergistic intensification of the action of a compound of the Formula according to the present invention.

Although it is known that certain cooling agents can reduce susceptibility to itching and thereby exert an alleviating effect (J.D. Bernhardt; Itch - Mechanisms and Management of Pruritus, McGraw-Hill Inc., 1994, ISBN 0-07-004935-1; B. Bromm et al., Neuroscience Letters Vol. 187, pp 157-160, 1995), there are no disclosures relating to the combination of cooling agents with the compound of the Formula.

Individual preferred cooling agents for use within the framework of the present invention are listed below. Those skilled in the art can add a large number of other cooling agents to this list; the cooling agents listed can also be used in combination with one another: I-menthol, d-menthol, racemic menthol, menthone glycerol acetal (trade name: Frescolat^{®} MGA), menthyl lactate (trade name: Frescolat^{®} ML; menthyl lactate is preferably I-menthyl lactate, especially I-menthyl I-lactate), substituted menthyl-3-carboxamides (e.g. menthyl-3-carboxylic acid N-ethylamide), 2-isopropyl-N-2,3-trimethylbutanamide, substituted cyclohexanecarboxamides, 3-menthoxypropane-1,2-diol, 2-hydroxyethyl menthyl carbonate, 2-hydroxypropyl menthyl carbonate, N-acetylglycine menthyl ester, isopulegol, hydroxycarboxylic acid menthyl esters (e.g. menthyl 3-hydroxy-butyrate), monomenthyl succinate, 2-mercaptocyclodecanone, menthyl 2-pyrrolidin-5-onecarboxylate, 2,3-dihydroxy-p-menthane, 3,3,5-trimethylcyclo-hexanone glycerol ketal, 3-menthyl-3,6-di- and trioxaalkanoates, 3-menthyl methoxyacetate and icilin.

Cooling agents that are preferred on the basis of their particular synergistic effect are I-menthol, d-menthol, racemic menthol, menthone glycerol acetal (trade name: Frescolat^{®} MGA), menthyl lactate (preferably I-menthyl lactate, especially I-menthyl I-lactate (trade name: Frescolat^{®} ML)), substituted menthyl-3-carboxamides (e.g. menthyl-3-carboxylic acid N-ethylamide), 2-isopropyl-N-2,3-trimethylbutanamide, substituted cyclohexanecarboxamides, 3-menthoxy-propane-1,2-diol, 2-hydroxyethyl menthyl carbonate, 2-hydroxypropyl menthyl carbonate and isopulegol.

Particularly preferred cooling agents are I-menthol, racemic menthol, menthone glycerol acetal (trade name: Frescolat^{®} MGA), menthyl lactate (preferably I-menthyl lactate, especially l-menthyl I-lactate (trade name: Frescolat^{®} ML)), 3-menthoxypropane-1,2-diol, 2-hydroxyethyl menthyl carbonate and 2-hydroxy-propyl menthyl carbonate.

Very particularly preferred cooling agents are I-menthol, menthone glycerol acetal (trade name: Frescolat^{®} MGA) and menthyl lactate (preferably I-menthyl lactate, especially I-menthyl l-lactate (trade name: Frescolat^{®} ML)).

The mixtures according to the invention, especially those identified as preferred, have a synergistically intensified efficacy against itching and/or in the reduction of skin reddening. The efficacy of mixtures according to the invention is superior to a surprising extent to that of products comprising exclusively a compound of the Formula (as indicated above) or exclusively a cooling agent.

The mixtures according to the invention are particularly effective and furthermore are free of any toxicologically or dermatologically critical secondary components; they can therefore be used without problems in pharmaceutical or cosmetic products. In general, it is pointed out that, in the concentration range relevant to efficacy, the substances to be used in cosmetic and/or pharmaceutical products
- should be toxicologically safe,
- should have a good skin tolerability,
- should be stable (especially in the conventional cosmetic and/or pharmaceutical formulations),
- should preferably be odourless and
- should be inexpensive to prepare (i.e. by using standard processes and/or by starting from standard precursors).

These requirements are met by the mixtures according to the invention.

Although the use concentration of the compound of the Formula to be used according to the invention can range from 0.0001 to 10 percent by mass - depending on the substance - as is already the case according to WO 2004/047833, it is preferable to use a low concentration of the compound of the Formula. A concentration range of 0.001 to 1 percent by mass is particularly preferred and a range of 0.01 to 0.2 percent by mass is very particularly preferred, based in each case on the total weight of a ready-to-use cosmetic or pharmaceutical end product.

Depending on the substance, the use concentration of the cooling agents to be used according to the invention ranges preferably from 0.01 to 20 percent by mass and particularly preferably from 0.1 to 5 percent by mass, based on the total weight of a ready-to-use cosmetic or pharmaceutical end product.

Particularly preferred mixtures according to the invention are those in which the mass ratio of the total amount of the compound of the Formula to the total amount of cooling agents ranges from 1:100 to 1:2, preferably from 1:50 to 1:5 and particularly preferably from 1:30 to 1:10. Thus the proportion by weight of cooling agents is preferably predominant compared with that of the compound of the Formula.

The mixtures according to the invention can be combined with a large number of other components to give preferred cosmetic and/or pharmaceutical mixtures or products.

### Combination with skin moisture regulators:

Itching occurs with particular intensity especially when the skin is dry. The use of skin moisture regulators in cosmetic and pharmaceutical products can significantly alleviate itching. The synergistically effective combinations according to the invention of the compound of the Formula (anthranilic acid amide) and cooling agents can therefore also be combined particularly advantageously with skin moisture regulators. Cosmetic preparations containing a mixture according to the invention can therefore advantageously also contain the following moisture retention regulators: sodium lactate, urea, urea derivatives, alcohols, glycerol, diols such as propylene glycol, hexylene glycol, 1,2-pentanediol, 1,2-hexanediol, 1,2-heptanediol, 1,2-octanediol, 1,2-nonanediol, 1,2-decanediol or mixtures of said diols, especially mixtures of 1,2-hexanediol and 1,2-octanediol, collagen, elastin or hyaluronic acid, diacyl adipates, petrolatum, urocanic acid, lecithin, panthenol, phytantriol, lycopene, (pseudo-)ceramides, glycosphingolipids, cholesterol, phytosterols, chitosan, chondroitin sulphate, lanolin, lanolin esters, amino acids, alpha-hydroxy acids (e.g. citric acid, lactic acid, malic acid) and derivatives thereof, mono-, di- and oligosaccharides such as glucose, galactose, fructose, mannose, fructose and lactose, polysugars such as ß-glucans, especially 1,3-1,4-ß-glucan from oats, alpha-hydroxy fatty acids, triterpene acids such as betulinic acid or ursolic acid, and algae extracts.

Depending on the substance, the use concentration of the moisture retention regulators ranges from 0.1 to 10 % (m/m) and preferably from 0.5 to 5 % (m/m), based on the total weight of a ready-to-use cosmetic or pharmaceutical end product. These data apply especially to diols that are advantageously to be used, such as hexylene glycol, 1,2-pentanediol, 1,2-hexanediol, 1,2-octanediol and 1,2-decanediol, as well as mixtures of 1,2-hexanediol and 1,2-octanediol.

### Combination with osmolytes:

The mixtures according to the invention can also be used together with osmolytes. Examples of osmolytes which may be mentioned are substances from the group comprising sugar alcohols (myoinositol, mannitol, sorbitol), quaternary amines such as taurine, choline, betaine, betaine glycine, ectoin, diglycerol phosphate, phosphorylcholine or glycerophosphorylcholines, amino acids such as glutamine, glycine, alanine, glutamate, aspartate or proline, phosphatidylcholine, phosphatidylinositol, inorganic phosphates, and polymers of said compounds, such as proteins, peptides, polyamino acids and polyols. All osmolytes have a skin moisturising action at the same time.

### Combination with nurturing substances:

In formulations containing mixtures according to the invention for the topical cosmetic or pharmaceutical treatment of e.g. dry, itchy skin, a high proportion especially of nurturing substances is also of particular advantage because of the reduced transepidermal water loss due to lipophilic components. In one preferred embodiment the compositions contain one or more nurturing animal and/or vegetable fats and oils such as olive oil, sunflower oil, refined soya oil, palm oil, sesame oil, rapeseed oil, almond oil, borage oil, evening primrose oil, coconut oil, shea butter, jojoba oil, sperm oil, tallow, neatsfoot oil and lard, and optionally other nurturing components such as fatty alcohols having 8 - 30 C atoms. The fatty alcohols used here can be saturated or unsaturated and linear or branched.

Nurturing substances which can particularly preferably be combined with the mixtures according to the invention also include especially
- ceramides, which are understood as meaning N-acylsphingosines (fatty acid amides of sphingosine) or synthetic analogues of such lipids (so-called pseudo-ceramides) that markedly improve the water retention capacity of the stratum corneum;
- phospholipids, e.g. soya lecithin, egg lecithin and cephalins; and
- petrolatum, paraffin oils and silicone oils, the latter including, inter alia, dialkyl- and alkylarylsiloxanes such as dimethylpolysiloxane and methylphenylpolysiloxane, and their alkoxylated and quaternised derivatives.

### Combination with preservatives, antiperspirants or chelators:

Cosmetic preparations containing mixtures according to the invention can also contain active compounds for preserving cosmetic products, antiperspirants and (metal) chelators.

### Combination with animal and/or vegetable protein hydrolysates:

Animal and/or vegetable protein hydrolysates can also advantageously be added to the mixtures according to the invention. The following are particularly advantageous in this context: fractions of elastin, collagen, keratin, lactalbumin, soya protein, oat protein, pea protein, almond protein and wheat protein, or corresponding protein hydrolysates, and also their condensation products with fatty acids, as well as quaternised protein hydrolysates, the use of vegetable protein hydrolysates being preferred.

### Combination with solvents:

If a cosmetic or dermatological preparation containing synergistically effective combinations of anthranilic acid amide and cooling agents is a solution or lotion, the following solvents can be used:
- water or aqueous solutions;
- fatty oils, fats, waxes and other natural and synthetic fatty substances, preferably esters of fatty acids with alcohols of low C number, e.g. with isopropanol, propylene glycol or glycerol, or esters of fatty alcohols with alkanoic acids of low C number or with fatty acids;
- alcohols, diols or polyols of low C number and their ethers, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products.

Mixtures of the abovementioned solvents are used in particular. Water can be an additional component of alcoholic solvents.

### Combination with other active compounds:

Cosmetic preparations containing a mixture according to the invention can also particularly advantageously contain anti-inflammatory compounds and/or compounds that alleviate reddening and/or other compounds that alleviate itching, it being possible to use any anti-inflammatory compounds and/or compounds that alleviate reddening and/or itching which are suitable or conventionally used for cosmetic and/or dermatological applications. Steroidal anti-inflammatory substances of the corticosteroid type, e.g. hydrocortisone, hydrocortisone derivatives such as hydrocortisone 17-butyrate, dexamethasone, dexamethasone phosphate, methylprednisolone or cortisone, are advantageously used as anti-inflammatory compounds or compounds that alleviate reddening and/or itching; other steroidal anti-inflammatories can be added to the list. It is also possible to use non-steroidal anti-inflammatories. Examples which should be mentioned here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen or benoxaprofen; or pyrazoles such as phenylbutazone, oxyphenylbutazone, febrazone or azapropazone. A possible alternative is to use natural anti-inflammatory substances or substances that alleviate reddening and/or itching. Plant extracts, special high-activity plant extract fractions and high-purity active substances isolated from plant extracts can be used. Particular preference is afforded to extracts, fractions and active substances from camomile, Aloe vera, Commiphora species, Rubia species, willow, willow-herb, oats, calendula, arnica, St John's wort, honeysuckle, rosemary, Passiflora incarnata, witch hazel, ginger or Echinacea, and pure substances such as, inter alia, bisabolol, apigenin, apigenin-7-glucoside, boswellic acid, phytosterols, glycyrrhizin, glabridin and licochalcone A. The synergistically effective combinations of anthranilic acid amide and cooling agents can also contain mixtures of two or more anti-inflammatory compounds.

Depending on the substance, the use concentration of the anti-inflammatory compounds which can be used ranges from 0.005 to 2 % (m/m) and preferably from 0.05 to 0.5 % (m/m), based on the total weight of a ready-to-use cosmetic or pharmaceutical end product. These data apply especially to bisabolol. Combination with antioxidants:
Cosmetic preparations containing a mixture according to the invention can also contain antioxidants, it being possible to use any antioxidants which are suitable or conventionally used for cosmetic and/or dermatological applications.

### Combination with vitamins:

Cosmetic preparations containing a mixture according to the invention can also contain vitamins and vitamin precursors, it being possible to use any vitamins or vitamin precursors which are suitable or conventionally used for cosmetic and/or dermatological applications.

### Combination with skin lighteners:

Cosmetic preparations containing a mixture according to the invention can also contain compounds with a skin lightening action, it being possible according to the invention to use any skin lightening compounds which are suitable or conventionally used for cosmetic and/or dermatological applications. Advantageous skin lightening compounds in this context are kojic acid, hydroquinone, arbutin, ascorbic acid, magnesium ascorbyl phosphate, 4-substituted resorcinol derivatives, liquorice root extracts and their glabridin or licochalcone A components, or extracts of Rumex and Ramulus species, extracts of pine species (Pinus) or extracts of Vitis species which contain skin lightening stilbene derivatives, inter alia.

### Combination with skin tanning agents:

Cosmetic preparations containing a mixture according to the invention can also contain compounds with a skin tanning action, it being possible in this context to use any skin tanning compounds which are suitable or conventionally used for cosmetic and/or dermatological applications. An example which may be mentioned here is dihydroxyacetone (DHA; 1,3-dihydroxy-2-propanone). DHA can exist in both monomeric and dimeric form, the proportion of dimer being predominant in the crystalline form.

### Combination with saccharides:

Cosmetic preparations containing a mixture according to the invention can also contain mono-, di- and oligosaccharides, e.g. glucose, galactose, fructose, mannose, fructose and lactose.

### Combination with plant extracts:

Cosmetic preparations containing a mixture according to the invention can also contain plant extracts, which are conventionally prepared by extraction of the whole plant or, in specific cases, exclusively from the blossom and/or leaves, wood, bark or roots of the plant.

### Combination with surfactants:

Cosmetic preparations containing a mixture according to the invention can also contain anionic, cationic, non-ionic and/or amphoteric surfactants, especially when crystalline or microcrystalline solids, e.g. inorganic micropigments, are to be incorporated into the preparations. Surfactants are amphiphilic substances capable of solubilising organic, non-polar substances in water. The hydrophilic parts of a surfactant molecule are usually polar functional groups, e.g. -COO⁻, -OSO₃²⁻ or -SO₃⁻, while the hydrophobic parts are normally non-polar hydrocarbon radicals. Surfactants are generally classified according to the type and charge of the hydrophilic part of the molecule. They can be divided into four groups:
- anionic surfactants,
- cationic surfactants,
- amphoteric surfactants and
- non-ionic surfactants.

Anionic surfactants normally contain carboxylate, sulphate or sulphonate groups as functional groups. In aqueous solution they form negatively charged organic ions in an acidic or neutral medium. Cationic surfactants are characterised virtually exclusively by the presence of a quaternary ammonium group. In aqueous solution they form positively charged organic ions in an acidic or neutral medium. Amphoteric surfactants contain both anionic and cationic groups and accordingly behave like anionic or cationic surfactants in aqueous solution, depending on the pH. They have a positive charge in a strongly acidic medium and a negative charge in an alkaline medium. In the neutral pH range, on the other hand, they are zwitterionic. Polyether chains are typical of non-ionic surfactants. Non-ionic surfactants do not form ions in an aqueous medium.

### A. Anionic surfactants

Anionic surfactants that can advantageously be used are acylamino acids (and salts thereof) such as
- acylglutamates, e.g. sodium acylglutamate, di-TEA palmitoylaspartate and sodium caprylic/capric glutamate,
- acylpeptides, e.g. palmitoyl-hydrolysed lactoprotein, sodium cocoyl-hydrolysed soya protein and sodium/potassium cocoyl-hydrolysed collagen,
- sarcosinates, e.g. myristoyl sarcosine, TEA lauroylsarcosinate, sodium lauroylsarcosinate and sodium cocoylsarcosinate,
- taurates, e.g. sodium lauroyltaurate and sodium methylcocoyltaurate,
- acyllactylates, lauroyllactylate and caproyllactylate,
- alaninates;
carboxylic acids and derivatives, such as lauric acid, aluminium stearate, magnesium alkanolate and zinc undecylenate,
- ester-carboxylic acids, e.g. calcium stearoyllactylate, laureth-6 citrate and sodium PEG-4 lauramidocarboxylate,
- ether-carboxylic acids, e.g. sodium laureth-13 carboxylate and sodium PEG-6 cocamidocarboxylate;
phosphoric acid esters and salts, such as DEA oleth-10 phosphate and di-laureth-4 phosphate;
sulphonic acids and salts, such as
- acylisethionates, e.g. sodium/ammonium cocoylisethionate,
- alkylarylsulphonates,
- alkylsulphonates, e.g. sodium coco monoglyceride sulphate, sodium C₁₂₋₁₄-olefinsulphonate, sodium laurylsulphoacetate and magnesium PEG-3 cocamidosulphate,
- sulphosuccinates, e.g. sodium dioctylsulphosuccinate, disodium laureth sulphosuccinate, disodium laurylsulphosuccinate and disodium MEA unde-cylenamidosulphosuccinate;
and
sulphuric acid esters such as
- alkyl ether sulphate, e.g. sodium, ammonium, magnesium, MIPA and TIPA laureth sulphate, sodium myreth sulphate and sodium C12-13 pareth 2sulphate,
- alkylsulphates, e.g. sodium, ammonium and TEA laurylsulphate.

### B. Cationic surfactants

Cationic surfactants that can advantageously be used are
- alkylamines,
- alkylimidazoles,
- ethoxylated amines and
- quaternary surfactants:
   RNH₂CH₂CH₂COO⁻ (at pH 7)
   RNHCH₂CH₂COO⁻ B⁺ (at pH 12), B⁺ = arbitrary cation, e.g. Na⁺
- esterquats

Quaternary surfactants contain at least one N atom that is covalently bonded to 4 alkyl or aryl groups. This produces a positive charge, irrespective of the pH.

Alkylbetaine, alkylamidopropylbetaine and alkylamidopropylhydroxysulfaine are advantageous. The cationic surfactants used can also preferably be selected from the group comprising quaternary ammonium compounds, in particular benzyltrialkylammonium chlorides or bromides, e.g. benzyldimethylstearylammonium chloride, and also alkyltrialkylammonium salts, e.g. cetyltrimethylammonium chloride or bromide, alkyldimethylhydroxyethylammonium chlorides or bromides, dialkyldimethylammonium chlorides or bromides, alkylamidoethyltrimethylammonium ether sulphates, alkylpyridinium salts, e.g. lauryl- or cetylpyrimidinium chloride, imidazoline derivatives and compounds of a cationic nature, such as amine oxides, e.g. alkyldimethylamine oxides or alkylaminoethyldimethylamine oxides. Cetyltrimethylammonium salts can be used particularly advantageously.

### C. Amphoteric surfactants

Amphoteric surfactants that can advantageously be used are
- acyl-/dialkylethylenediamine, e.g. sodium acylamphoacetate, disodium acylamphodipropionate, disodium alkylamphodiacetate, sodium acylamphohydroxypropylsulphonate, disodium acylamphodiacetate and sodium acylamphopropionate,
- N-alkylamino acids, e.g. aminopropylalkylglutamide, alkylaminopropionic acid, sodium alkylimidodipropionate and lauroamphocarboxyglycinate.

### D. Non-ionic surfactants

Non-ionic surfactants that can advantageously be used are
- alcohols,
- alkanolamides such as cocamides MEA/DEA/MIPA,
- amine oxides such as cocamidopropylamine oxide,
- esters formed by the esterification of carboxylic acids with ethylene oxide, glycerol, sorbitan or other alcohols,
- ethers, e.g. ethoxylated/propoxylated alcohols, ethoxylated/propoxylated esters, ethoxylated/propoxylated glycerol esters, ethoxylated/propoxylated cholesterols, ethoxylated/propoxylated triglyceride esters, ethoxylated/ propoxylated lanolin, ethoxylated/propoxylated polysiloxanes, propoxylated POE ethers, and alkyl polyglycosides such as lauryl glucoside, decyl glycoside and coco glycoside,
- sucrose esters and ethers,
- polyglycerol esters, diglycerol esters and monoglycerol esters,
- methyl glucose esters and esters of hydroxy acids.

The use of a combination of anionic and/or amphoteric surfactants with one or more non-ionic surfactants is also advantageous.

The surface-active substance can be present in a concentration of between 1 and 98 % (m/m) in the preparations containing synergistically effective combinations of anthranilic acid amides and cooling agents, based on the total weight of the preparations.

### Emulsions comprising a mixture according to the invention:

Cosmetic or dermatological preparations containing synergistically effective combinations according to the invention of anthranilic acid amide and cooling agents can also take the form of emulsions.

The oily phase can advantageously be selected from the following group of substances:
- mineral oils and mineral waxes;
- fatty oils, fats, waxes and other natural and synthetic fatty substances, preferably esters of fatty acids with alcohols of low C number, e.g. with isopropanol, propylene glycol or glycerol, or esters of fatty alcohols with alkanoic acids of low C number or with fatty acids;
- alkyl benzoates;
- silicone oils such as dimethylpolysiloxanes, diethylpolysiloxanes, diphenylpolysiloxanes and mixed forms thereof.
   (a) Esters of saturated and/or unsaturated, branched and/or unbranched alkanecarboxylic acids having a chain length of 3 to 30 C atoms and saturated and/or unsaturated, branched and/or unbranched alcohols having a chain length of 3 to 30 C atoms, and (b) esters of aromatic carboxylic acids and saturated and/or unsaturated, branched and/or unbranched alcohols having a chain length of 3 to 30 C atoms, can advantageously be used. Preferred ester oils are isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 3,5,5-trimethylhexyl 3,5,5-trimethylhexanoate, 2-ethylhexyl isononanoate, 2-ethylhexyl 3,5,5-trimethylhexanoate, 2-ethylhexyl 2-ethylhexanoate, 2-ethylhexyl palmitate, 2-ethylhexyl laurate, 2-hexyldecyl stearate, 2-octyldodecyl palmitate, oleyl oleate, oleyl erucate, erucyl oleate, erucyl erucate and synthetic, semisynthetic and natural mixtures of such esters, e.g. jojoba oil.

Furthermore, the oily phase can advantageously be selected from the group comprising branched and unbranched hydrocarbons and waxes, silicone oils, dialkyl ethers, the group comprising saturated or unsaturated, branched or unbranched alcohols, and also fatty acid triglycerides, specifically the triglycerol esters of saturated and/or unsaturated, branched and/or unbranched alkanecarboxylic acids having a chain length of 8 to 24 and especially 12 to 18 C atoms. The fatty acid triglycerides can advantageously be selected from the group comprising synthetic, semisynthetic and natural oils, e.g. olive oil, sunflower oil, soya oil, groundnut oil, rapeseed oil, almond oil, palm oil, coconut oil, palm kernel oil and the like. Arbitrary mixtures of such oil and wax components can also advantageously be used. In some cases it is also advantageous to use waxes, e.g. cetyl palmitate, as the sole lipid component of the oily phase; advantageously, the oily phase is selected from the group comprising 2-ethylhexyl isostearate, octyldodecanol, isotridecyl isononanoate, isoeicosane, 2-ethylhexyl cocoate, C₁₂₋₁₅-alkyl benzoate, caprylic/capric triglyceride and dicaprylyl ether. Mixtures of C₁₂₋₁₅-alkyl benzoate and 2-ethylhexyl isostearate, mixtures of C₁₂₋₁₅-alkyl benzoate and isotridecyl isononanoate and mixtures of C₁₂₋₁₅-alkyl benzoate, 2-ethylhexyl isostearate and isotridecyl isononanoate are particularly advantageous. The hydrocarbons paraffin oil, squalane and squalene can also advantageously be used. Advantageously, the oily phase can further contain cyclic or linear silicone oils or consist entirely of such oils, although it is preferable to use other oily phase components in addition to the silicone oil(s). Cyclomethicone (e.g. decamethylcyclopentasiloxane) can advantageously be used as a silicone oil. However, other silicone oils can also advantageously be used, examples being undecamethylcyclotrisiloxane, polydimethylsiloxane and poly(methylphenylsiloxane). Furthermore, mixtures of cyclomethicone and isotridecyl isononanoate and of cyclomethicone and 2-ethylhexyl isostearate are particularly advantageous.

The aqueous phase of preparations that contain synergistically effective combinations of anthranilic acid amide and cooling agents and take the form of an emulsion can advantageously comprise alcohols, diols or polyols of low C number, as well as ethers thereof, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products, and also alcohols of low C number, e.g. ethanol, isopropanol, 1,2-propanediol and glycerol, and in particular one or more thickeners, which can advantageously be selected from the group comprising silicon dioxide, aluminium silicates, polysaccharides and derivatives thereof, e.g. hyaluronic acid, xanthan gum, hydroxypropyl methyl cellulose, and particularly advantageously from the group comprising polyacrylates, preferably a polyacrylate from the group comprising the so-called carbopols, e.g. carbopols of types 980, 981, 1382, 2984 and 5984, in each case on their own or in combination.

Preparations that contain synergistically effective combinations of anthranilic acid amide and cooling agents and take the form of an emulsion advantageously comprise one or more emulsifiers. O/W emulsifiers can, for example, advantageously be selected from the group comprising polyethoxylated or polypropoxylated or polyethoxylated and polypropoxylated products, e.g.:
- fatty alcohol ethoxylates,
- ethoxylated wool wax alcohols,
- polyethylene glycol ethers of the general formula R-O-(-CH₂-CH₂-O-)ₙ-R',
- fatty acid ethoxylates of the general formula R-COO-(-CH₂-CH₂-O-)ₙ-H,
- etherified fatty acid ethoxylates of the general formula

   R-COO-(-CH₂-CH₂-O-)ₙ-R',
- esterified fatty acid ethoxylates of the general formula

   R-COO-(-CH₂-CH₂-O-)ₙ-C(O)-R',
- polyethylene glycol glycerol fatty acid esters,
- ethoxylated sorbitan esters,
- cholesterol ethoxylates,
- ethoxylated triglycerides,
- alkyl ether carboxylic acids of the general formula

   R-COO-(-CH₂-CH₂-O-)ₙ-OOH,

   where n is a number from 5 to 30,
- polyoxyethylene sorbitol fatty acid esters,
- alkyl ether sulphates of the general formula R-O-(-CH₂-CH₂-O-)ₙ-SO₃-H,
- fatty alcohol propoxylates of the general formula R-O-(-CH₂-CH(CH₃)-O-)ₙ-H,
- polypropylene glycol ethers of the general formula

   R-O-(-CH₂-CH(CH₃)-O-)ₙ-R',
- propoxylated wool wax alcohols,
- etherified fatty acid propoxylates R-COO-(-CH₂-CH(CH₃)-O-)ₙ-R',
- esterified fatty acid propoxylates of the general formula

   R-COO-(-CH₂-CH(CH₃)-O-)ₙ-C(O)-R',
- fatty acid propoxylates of the general formula R-COO-(-CH₂-CH(CH₃)-O-)ₙ-H,
- polypropylene glycol glycerol fatty acid esters,
- propoxylated sorbitan esters,
- cholesterol propoxylates,
- propoxylated triglycerides,
- alkyl ether carboxylic acids of the general formula

   R-O-(-CH₂-CH(CH₃)-O-)ₙ-CH₂-COOH,
- alkyl ether sulphates and the acids on which these sulphates are based of the general formula

   R-O-(-CH₂-CH(CH₃)-O-)ₙ-SO₃-H,
- fatty alcohol ethoxylates/propoxylates of the general formula R-O-Xₙ-Yₘ-
- polypropylene glycol ethers of the general formula R-O-Xₙ-Yₘ-R',
- etherified fatty acid propoxylates of the general formula R-COO-Xₙ-Yₘ-R',
- fatty acid ethoxylates/propoxylates of the general formula R-COO-Xₙ-Yₘ-H.

According to the invention, the polyethoxylated or polypropoxylated or polyethoxylated and polypropoxylated O/W emulsifiers used are particularly advantageously selected from the group comprising substances having HLB values of 11 - 18, very particularly advantageously having HLB values of 14.5 -15.5, if the O/W emulsifiers contain saturated radicals R and R'. If the O/W emulsifiers contain unsaturated radicals R and/or R', or if isoalkyl derivatives are present, the preferred HLB value of such emulsifiers can also be lower or higher.

It is advantageous to select the fatty alcohol ethoxylates from the group comprising ethoxylated stearyl alcohols, cetyl alcohols and cetylstearyl alcohols (cetearyl alcohols). The following are particularly preferred:
polyethylene glycol (13) stearyl ether (steareth-13), polyethylene glycol (14) stearyl ether (steareth-14), polyethylene glycol (15) stearyl ether (steareth-15), polyethylene glycol (16) stearyl ether (steareth-16), polyethylene glycol (17) stearyl ether (steareth-17), polyethylene glycol (18) stearyl ether (steareth-18), polyethylene glycol (19) stearyl ether (steareth-19), polyethylene glycol (20) stearyl ether (steareth-20), polyethylene glycol (12) isostearyl ether (isosteareth-12), polyethylene glycol (13) isostearyl ether (isosteareth-13), polyethylene glycol (14) isostearyl ether (isosteareth-14), polyethylene glycol (15) isostearyl ether (isosteareth-15), polyethylene glycol (16) isostearyl ether (isosteareth-16), polyethylene glycol (17) isostearyl ether (isosteareth-17), polyethylene glycol (18) isostearyl ether (isosteareth-18), polyethylene glycol (19) isostearyl ether (isosteareth-19), polyethylene glycol (20) isostearyl ether (isosteareth-20), polyethylene glycol (13) cetyl ether (ceteth-13), polyethylene glycol (14) cetyl ether (ceteth-14), polyethylene glycol (15) cetyl ether (ceteth-15), polyethylene glycol (16) cetyl ether (ceteth-16), polyethylene glycol (17) cetyl ether (ceteth-17), polyethylene glycol (18) cetyl ether (ceteth-18), polyethylene glycol (19) cetyl ether (ceteth-19), polyethylene glycol (20) cetyl ether (ceteth-20), polyethylene glycol (13) isocetyl ether (isoceteth-13), polyethylene glycol (14) isocetyl ether (isoceteth-14), polyethylene glycol (15) isocetyl ether (isoceteth-15), polyethylene glycol (16) isocetyl ether (isoceteth-16), polyethylene glycol (17) isocetyl ether (isoceteth-17), polyethylene glycol (18) isocetyl ether (isoceteth-18), polyethylene glycol (19) isocetyl ether (isoceteth-19), polyethylene glycol (20) isocetyl ether (isoceteth-20), polyethylene glycol (12) oleyl ether (oleth-12), polyethylene glycol (13) oleyl ether (oleth-13), polyethylene glycol (14) oleyl ether (oleth-14), polyethylene glycol (15) oleyl ether (oleth-15), polyethylene glycol (12) lauryl ether (laureth-12), polyethylene glycol (12) isolauryl ether (isolaureth-12), polyethylene glycol (13) cetylstearyl ether (ceteareth-13), polyethylene glycol (14) cetylstearyl ether (ceteareth-14), polyethylene glycol (15) cetylstearyl ether (ceteareth-15), polyethylene glycol (16) cetylstearyl ether (ceteareth-16), polyethylene glycol (17) cetylstearyl ether (ceteareth-17), polyethylene glycol (18) cetylstearyl ether (ceteareth-18), polyethylene glycol (19) cetylstearyl ether (ceteareth-19) and polyethylene glycol (20) cetylstearyl ether (ceteareth-20).

It is also advantageous to select the fatty acid ethoxylates from the following group:
polyethylene glycol (20) stearate, polyethylene glycol (21) stearate, polyethylene glycol (22) stearate, polyethylene glycol (23) stearate, polyethylene glycol (24) stearate, polyethylene glycol (25) stearate, polyethylene glycol (12) isostearate, polyethylene glycol (13) isostearate, polyethylene glycol (14) isostearate, polyethylene glycol (15) isostearate, polyethylene glycol (16) isostearate, polyethylene glycol (17) isostearate, polyethylene glycol (18) isostearate, polyethylene glycol (19) isostearate, polyethylene glycol (20) isostearate, polyethylene glycol (21) isostearate, polyethylene glycol (22) isostearate, polyethylene glycol (23) isostearate, polyethylene glycol (24) isostearate, polyethylene glycol (25) isostearate, polyethylene glycol (12) oleate, polyethylene glycol (13) oleate, polyethylene glycol (14) oleate, polyethylene glycol (15) oleate, polyethylene glycol (16) oleate, polyethylene glycol (17) oleate, polyethylene glycol (18) oleate, polyethylene glycol (19) oleate and polyethylene glycol (20) oleate.

Sodium laureth-11 carboxylate can advantageously be used as an ethoxylated alkyl ether carboxylic acid or a salt thereof. Sodium laureth 1-4 sulphate can advantageously be used as an alkyl ether sulphate. Polyethylene glycol (30) cholesteryl ether can advantageously be used as an ethoxylated cholesterol derivative. Polyethylene glycol (25) soyasterol has also proved useful.

Polyethylene glycol (60) evening primrose glycerides can advantageously be used as ethoxylated triglycerides.

It is also advantageous to select the polyethylene glycol glycerol fatty acid esters from the group comprising polyethylene glycol (20) glyceryl laurate, polyethylene glycol (21) glyceryl laurate, polyethylene glycol (22) glyceryl laurate, polyethylene glycol (23) glyceryl laurate, polyethylene glycol (6) glyceryl caprylate/caprate, polyethylene glycol (20) glyceryl oleate, polyethylene glycol (20) glyceryl isostearate and polyethylene glycol (18) glyceryl oleate/cocoate.

It is likewise favourable to select the sorbitan esters from the group comprising polyethylene glycol (20) sorbitan monolaurate, polyethylene glycol (20) sorbitan monostearate, polyethylene glycol (20) sorbitan monoisostearate, polyethylene glycol (20) sorbitan monopalmitate and polyethylene glycol (20) sorbitan monooleate.

The following can be used as advantageous W/O emulsifiers: fatty alcohols having 8 to 30 carbon atoms, monoglycerol esters of saturated and/or unsaturated, branched and/or unbranched alkanecarboxylic acids having a chain length of 8 to 24, in particular 12 to 18 C atoms, diglycerol esters of saturated and/or unsaturated, branched and/or unbranched alkanecarboxylic acids having a chain length of 8 to 24, in particular 12 to 18 C atoms, monoglycerol ethers of saturated and/or unsaturated, branched and/or unbranched alcohols having a chain length of 8 to 24, in particular 12 to 18 C atoms, diglycerol ethers of saturated and/or unsaturated, branched and/or unbranched alcohols having a chain length of 8 to 24, in particular 12 to 18 C atoms, propylene glycol esters of saturated and/or unsaturated, branched and/or unbranched alkanecarboxylic acids having a chain length of 8 to 24, in particular 12 to 18 C atoms, and sorbitan esters of saturated and/or unsaturated, branched and/or unbranched alkanecarboxylic acids having a chain length of 8 to 24, in particular 12 to 18 C atoms.

Particularly advantageous W/O emulsifiers are glyceryl monostearate, glyceryl monoisostearate, glyceryl monomyristate, glyceryl monooleate, diglyceryl monostearate, diglyceryl monoisostearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, sucrose distearate, cetyl alcohol, stearyl alcohol, arachidyl alcohol, behenyl alcohol, isobehenyl alcohol, selachyl alcohol, chimyl alcohol, polyethylene glycol (2) stearyl ether (steareth-2), glyceryl monolaurate, glyceryl monocaprate and glyceryl monocaprylate.

### Preferred formulations:

The mixtures according to the invention can be incorporated without difficulty into conventional cosmetic, dermatological/keratological or pharmaceutical formulations such as, inter alia, pump sprays, aerosol sprays, creams, shampoos, facial cleaners, deodorants, ointments, tinctures, lotions, nail care products (e.g. nail varnishes, nail varnish removers, nail balsams) and the like. In this context it is also possible, and in some cases advantageous, to combine the synergistically effective combinations of anthranilic acid amide and cooling agents with other active compounds. In this context the cosmetic and/or dermatological/keratological formulations containing synergistically effective combinations of anthranilic acid amide and cooling agents can otherwise be of conventional composition and be used for treatment of the skin and/or hair in the sense of a dermatological/keratological treatment or a treatment in the sense of care cosmetics. However, the synergistically effective combinations of anthranilic acid amide and cooling agents can also be used in make-up products in decorative cosmetics.

### Combination with sunscreens:

For use, the cosmetic and/or dermatological/keratological formulations containing a mixture according to the invention are applied to the skin and/or hair in an adequate amount in the manner conventionally used for cosmetics and dermatological products. In this context cosmetic and dermatological preparations that contain a mixture according to the invention and additionally act as a sunscreen also offer particular advantages. Advantageously, these preparations contain at least one UVA filter and/or at least one UVB filter and/or at least one inorganic pigment. In this case the preparations can take various forms such as those conventionally employed for preparations of this type. Thus they can be e.g. a solution, an emulsion of the water-in-oil (W/O) type or of the oil-in-water (O/W) type or a multiple emulsion, e.g. of the water-in-oil-in-water (W/O/W) type, a gel, a hydrodispersion, a solid stick or else an aerosol.

### Combination with cosmetic auxiliaries:

In cosmetic preparations, the mixtures according to the invention can advantageously also be combined with cosmetic auxiliaries such as those conventionally used in such preparations, e.g. antioxidants, perfume oils, antifoams, colorants, pigments with a colouring action, thickeners, surface-active substances, emulsifiers, plasticisers, other moisturising and/or moisture-retaining substances, fats, oils, waxes or other conventional constituents of a cosmetic formulation, such as alcohols, polyols, polymers, foam stabilisers, electrolytes, organic solvents or silicone derivatives. According to the invention, any conceivable antioxidants, perfume oils, antifoams, colorants, pigments with a colouring action, thickeners, surface-active substances, emulsifiers, plasticisers, moisturising and/or moisture-retaining substances, fats, oils, waxes, alcohols, polyols, polymers, foam stabilisers, electrolytes, organic solvents or silicone derivatives that are suitable or conventionally used for cosmetic and/or dermatological applications can be used here.

Preferred (ready-to-use) cosmetic or pharmaceutical (end) products comprising a mixture according to the present invention are selected from the group consisting of:
soap, synthetic detergent, liquid washing, shower and bath preparation, solution, dispersion, suspension, cream, lotion, milk, W/O-, O/W- or multiple emulsion, PIT emulsion, emulsion foam, micro-emulsion, nano-emulsion, Pickering emulsion, ointment, paste, gel (including hydrogel, hydrodispersion gel, oleogel), oil, toner, balsam, serum, powder, eau de toilette, toilette, eau de cologne, perfume, wax, stick, roll-on, (pump) spray, nasal spray, nasal drops, aerosol (foaming, non-foaming or post-foaming), (O/W) day cream, (O/W) skin lotion, (W/O) night cream, body spray, suncreen lotion, after-sun product (balm, lotion, milk), foot care product (including keratolytics, deodorants), shaving foam, aftershave (balm, lotion), depilatory product, hair care product [e.g. shampoo (including 2-in-1 shampoo), conditioner, hair tonic, hair water, hair rinse, hair cream, pomade, perm and setting lotion, hair smoothing product (detangling product, relaxer), hair strengthener, styling aid (e.g. gel or wax), blonding product, hair dye (e.g. temporary hair dyes, colour rinses, semi-permanent and permanent hair dyes)], nail care products (e.g. nail polish and nail polish remover), deodorant, antiperspirant, mouthwash, (wet) wipe, suppository, ophthalmic preparation (e.g. drops, ointment), makeup, makeup remover, decorative cosmetics (e.g. powder, eyeshadows, kohl pencil, lipstick).

A mixture according to the invention is particularly advantageous when applied to sensitive skin and/or to treat redness or itching caused by mechanical stress, e.g. as caused by shaving. Shaving and after-shave products comprising anthranilic acid amides of Formula 1 alone (i.e. without the presence of a cooling agent) show good efficacy in terms of reduction of skin reddening and alleviating itching, but are remarkably improved when combined with one or more cooling agents.

Thus, in another preferred embodiment, shaving and after-shave products comprising a mixture according to the invention are applied to the skin, in particular for prevention and/or reduction of skin reddening and alleviating itching caused by shaving or experienced during shaving. The so-called razor burn thereby may be significantly reduced (see also examples S1 and S2).

Preferred shaving and after-shave products are shaving foam, shaving gel, shaving soap, shaving lather, after-shave balm, after-shave lotion, after-shave splash, and after-shave. These shaving and after-shave products advantageously may comprise additional (skin care) agents such as allantoin, panthenol, aloe vera, vitamins (such as vitamin E), antioxidants, facial cleaners and/or additional skin sootheners.

A mixture according to the invention is also particularly advantageous when applied to (human) nasal skin, nasal mucosa and/or the nasal cavity, e.g. in the form of nasal drops or a nasal spray, to treat skin or mucosa redness or itching (e.g. as caused by allergic stress or a bad cold). Nasal products comprising anthranilic acid amide of the Formula alone (i.e. without the presence of a cooling agent) show good efficacy in terms of reduction of skin or mucosa reddening and alleviating itching, but are remarkably improved when combined with one or more cooling agents.

Thus, in another preferred embodiment, nasal products (in particular nasal sprays, nasal drops) comprising a mixture according to the invention are applied to nasal skin, nasal mucosa and/or the nasal cavity, in particular for prevention and/or reduction of skin or mucosa reddening and alleviating itching. Nasal irritation, itching and redness thereby may be significantly reduced (see also examples N1 and N2). In addition such nasal products allow faster and better results than most oral products.

Preferably nasal sprays according to the present invention are topically administered to the nasal mucosa, for example by means of a metering, atomizing spray pump.

Preferred nasal products according to the present invention are nasal (allergy) spray, nasal drops and nasal ointments. Nasal products according to the invention advantageously may comprise additional agents and/or actives such as allantoin, panthenol, aloe vera, aloe barbadensis, glycerin, vitamins (such as vitamin E), antioxidants, benzalkonium chloride, (microcrystalline) cellulose, carboxymethylcellulose sodium, dextrose, citric acid, corticosteroids, and/or additional skin sootheners.

As regards other cosmetic and pharmaceutical active compounds, bases and auxiliaries which can particularly preferably be combined with the synergistically effective combinations according to the invention of anthranilic acid amide and cooling agents, reference may be made to the detailed descriptions in WO 2004/047833 and WO 03/069994.

### Combination with perfumes:

The mixtures according to the invention can also be used as a component of perfume compositions and, especially because of their specific activity, can impart an additional itch-alleviating or antiallergic property e.g. to a perfumed finished product. Particularly preferred perfume compositions comprise (a) a sensorially effective amount of a perfume, (b) an itch-regulating, antiallergic and/or hyposensitising amount of a synergistically effective mixture of anthranilic acid amide and cooling agents, and (c) optionally one or more excipients and/or additives. Since the proportion of perfume in a cosmetic finished product is frequently in the region of approx. 1 % (m/m), a perfume containing a compound of the Formula according to the invention will preferably contain about 0.1 - 10 % (m/m) of the Formula. It has proved particularly advantageous that the synergistically effective combinations of anthranilic acid amides and cooling agents have only a weak inherent odour or are even completely odourless, since this property predestines them in particular for use in a perfume composition.

Preferred embodiments of the mixtures according to the invention and of the processes and uses according to the invention can be seen from the following Examples and corresponding tables:

### Examples

The synergistic intensification of the itch-alleviating efficacy of the active compound combinations according to the invention is apparent from the human in vivo studies (skin prick test) described below.

Example 1: Human skin prick test for detecting the synergistically intensified efficacy of combinations consisting of an itch-alleviating compound (dihydroavenanthramide D; CARN: 697235-49-7; 2-[[3-(4-hydroxyphenyl)-1-oxopropyl]-amino]benzoic acid (9Cl)) and the cooling agent menthyl lactate (trade name: Frescolat® ML)

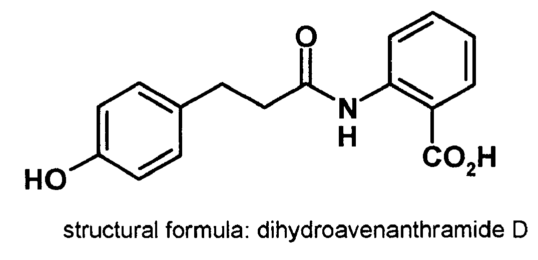

### Description of the test method:

The tests were carried out on 10 subjects (5 test areas on the inside forearm; 1 x untreated + 4 application areas for 4 samples).

### Samples:

1. Placebo formulation;
2. Product A: as placebo formulation but additionally containing 1 % (m/m) of menthyl lactate;
3. Product B: as placebo formulation but additionally containing 0.05 % (m/m) of dihydroavenanthramide D;
4. Product C: as placebo formulation but additionally containing 0.025 % (m/m) of dihydroavenanthramide D and 0.5 % (m/m) of Frescolat ML.

### Test procedure:

A defined amount of histamine chloride solution (HAL Allergie GmbH, Düsseldorf; concentration: 10 mg/ml) was applied to the skin of the inside forearm. The skin was then lightly scratched on the surface with a special lancet (Feather Safety Razor; LTD Medical Division, Japan). This produced intense itching within 5 minutes. The placebo formulation and Products A-C were then applied (amount: 2 mg/cm²). The effect of Products A-C on alleviation of the itching compared with the untreated area and the placebo was determined after 90'. The test procedure was carried out under standardised conditions (20 °C ± 1 %; humidity: 50 % ± 5%).

### Result:

1. Products A-C showed a significantly greater reduction of itching than the untreated area and the placebo formulation without active compound (Fig. 1).
2. Product C showed the greatest reduction of itching compared with Products A and B (Fig. 1).

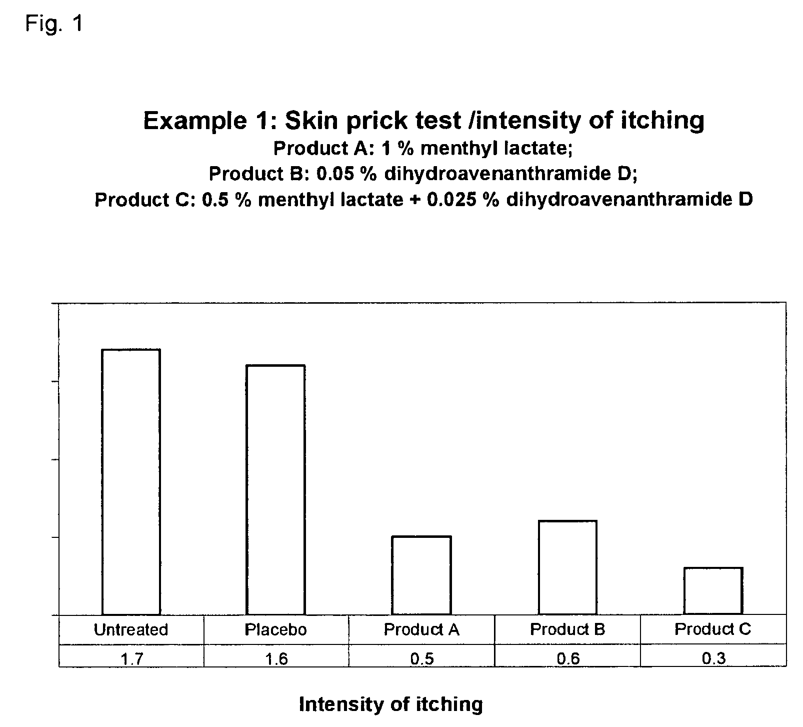

The human in vivo skin prick test study verifies by example that the combination of anthranilic acid amides of general Formula 1 with cooling agents achieves a greater reduction of itching. A significantly greater reduction of itching could be observed for Product C than for Products A and B (Fig. 1). The reduction of itching exceeded the values to be expected from a simple additive effect, thereby unambiguously proving a synergistic effect of Product C containing 0.5 % (m/m) of Frescolat ML and 0.025 % (m/m) of dihydroavenanthramide D. A synergistic intensification of the reduction of reddening by Product C compared with Products A and B could likewise be detected as part of the experiment.

The synergistic increase in efficacy afforded by the active compound combination according to the invention, consisting of a cooling agent and an itch-alleviating compound, can be detected on the basis of the present sensory data using Kull's equation (F.C. Kull et al.; Applied Microbiology Vol. 9, pp 538-541 (1961); David C. Steinberg; Cosmetics & Toiletries Vol. 115 (No. 11), pp 59-62; November 2000; for method of calculation see also Table 10). Kull's equation makes it possible to compare the pure substances and the active compound mixtures prepared therefrom in respect of their itch-alleviating efficacy. What is determined here is the so-called synergy index (SI), which constitutes a measure of the synergistic efficacy, but also the possibly antagonistic efficacy, of an itch-alleviating mixture. A synergistic effect is evident when the SI value found is less than 1. If the calculated SI is exactly 1, on the other hand, this indicates a purely additive effect of two itch-alleviating products. An SI value greater than 1 indicates a (frequently undesirable) antagonistic effect.

Calculation of the SI value for treatment with a mixture consisting of menthyl lactate and dihydroavenanthramide D after an incubation phase of 90 minutes is shown by way of example below. The calculated SI of 0.066 clearly indicates that the mixture consisting of menthyl lactate and dihydroavenanthramide D represents a strongly synergistic active compound combination.

**Table 1: Calculation of the synergy index (SI) of a menthyl lactate/dihydroavenanthramide D mixture (Product C) consisting of the reference cooling agent (A) and the reference itch-alleviating compound dihydroavenanthramide D (Product B)**

| | A | B | C |
|---|---|---|---|
| | Menthyl lactate (Frescolate ML) 1 % (m/m) | Dihydroavenanthramide D 0.05 % (m/m) | Menthyl lactate (0.5 % (m/m)) + Dihydroavenanthramide D (0.025 % (m/m)) |
| Alleviation of itching (intensity scale: 0 - 2) 2 = intense itching 0 = mild itching | 0.5 | 0.6 | 0.3 |
| | | | |
| Kull's equation: SI = CxD/A + CxE/B | | | |
| | | | |
| Alleviation of itching: product A | 0.5 | | |
| Alleviation of itching: product B | 0.6 | | |
| Alleviation of itching: product C | 0.3 | | |
| D: proportion of A in C | 0.5 | | |
| E: proportion of B in C | 0.5 | | |
| **SI: synergy index** | **0.55** | | |
| | | | |
| Literature: Synergy Index: | | | |
| D.C. Steinberg; Cosmetics & Toiletries 115 (11); pp 59-62 (2000) | | | |
| F.C. Kull et al.; Applied Microbiology 9; pp 538-541 (1961) | | | |

### Example 2: Formulations

Cosmetic formulations which are particularly suitable for alleviating itching and reducing reddening are listed by way of example below (Table 2).

### Key to formulations

**1 = O/W day cream**
**2 = O/W skin lotion, with plant extract**
**3 = after-sun balm**
**4 = body spray, for sensitive skin**
**5 = sunscreen lotion (O/W), broad spectrum protection**
**6 = W/O night cream**
**7 = shampoo**

**Table 2**

| **NAME OF MATERIAL (SUPPLIER)** | **INCI** | **% (M/M)** | | | | | | |
|---|---|---|---|---|---|---|---|---|
| | | **1** | **2** | **3** | **4** | **5** | **6** | **7** |
| -(-Alpha-)-bisabolol, natural (Symrise) | Bisabolol | | | 0.1 | | | 0.1 | |
| Abil 350 (Degussa-Goldschmidt) | Dimethicone | 0.5 | 2.0 | 1.0 | | | | |
| Allantoin (Merck) | Allantoin | | | 0.1 | | | | |
| Aloe vera gel concentrate 10/1 (Symrise) | Water (aqua), Aloe barbadensis leaf juice | | | 3.0 | | | 3.0 | |
| Alugel 34 TH (Baerlocher) | Aluminium stearate | | | | | | 1.0 | |
| Symatrix | Maltodextrin, Rubus fructicosus (blackberry) leaf extract | 0.3 | 0.1 | 1.0 | 0.1 | 0.3 | | |
| Butylene glycol | Butylene glycol | | | 5.0 | | | | |
| Carbopol ETD 2050 (Noveon) | Carbomer | | | | | 0.2 | | |
| Carbopol Ultrez-10 (Noveon) | Carbomer | | 0.1 | | | | | |
| Cetiol OE (Cognis) | Dicaprylyl ether | | | 4.0 | | | | |
| Cetiol SB 45 (Cognis) | Butyrospermum parkii (shea butter) | | | 1.0 | | | | |
| Citric acid 10% solution | Citric acid | | | | | | | 0.3 |
| Comperlan 100 (Cognis) | Cocamide MEA | | | | | | | 0.5 |
| Dihydroavenanthramide D (Symrise) | Formula 8 | 0.05 | 0.05 | 0.05 | 0.05 | 0.05 | 0.05 | 0.05 |
| Dow Coming 246 Fluid (Dow Corning) | Cyclohexasiloxane (and) cyclopentasiloxane | | | | | 2.0 | | |
| Dow Coming 345 Fluid (Dow Corning) | Cyclomethicone | | | | 0.5 | | | |
| D-panthenol (BASF) | Panthenol | | | 1.0 | | | | |
| Dracorin CE (Symrise) | Glyceryl stearate citrate | 5.0 | | | | | | |
| Dracorin GMS (Symrise) | Glyceryl stearate | | 2.0 | | | | | |
| Dracorin GOC (Symrise) | Glyceryl oleate citrate, caprylic/capric triglyceride | | | | 2.0 | | | |
| Drago-Beta-Glucan (Symrise) | Water (aqua), butylene glycol, glycerol, Avena sativa (oat) kernel extract | 0.3 | | | | | | |
| Dragocid Liquid (Symrise) | Phenoxyethanol, methylparaben, ethylparaben, butylparaben, propylparaben, isobutylparaben | | 0.80 | 0.70 | | 0.70 | 0.80 | |
| Dragoderm (Symrise) | Glycerol, Triticum vulgare (wheat) gluten, water (aqua) | | | | | | | 2.0 |
| Drago-Oat-Active (Symrise) | Water (aqua), butylene glycol, Avena sativa (oat) kernel extract | | | | 1.0 | | | |
| Dragosan W/O Liquid (Symrise) | Polyglyceryl 3-polyricinoleate, sorbitan isostearate | | | | | | 1.0 | |
| Dragosan W/O P (Symrise) | Sorbitan isostearate, hydrogenated castor oil, ceresin, beeswax (Cera alba) | | | | | | 6.0 | |
| Dragosantol (Symrise) | Bisabolol | | | | 0.1 | 0.1 | | |
| Dragoxat EH (Symrise) | Ethylhexyl ethylhexanoate | 3.0 | 3.0 | | 4.0 | | | |
| EDETA B Powder (BASF) | Tetrasodium EDTA | | | | | | | 0.1 |
| EDETA DB (BASF) | Disodium EDTA | | | | | 0.1 | | |
| Emulsiphos (Symrise) | Potassium cetyl phosphate, hydrogenated palm glycerides | | 2.0 | | | 1.5 | | |
| Ethanol 96 % | Ethanol | | | | | | | |
| Extrapon Green Tea GW (Symrise) | Glycerol, water (aqua), Camellia sinensis leaf extract | | 0.2 | | | | | |
| Extrapone Hamamelis Distillate, colourless (Symrise) | Propylene glycol, Hamamelis virginiana (witch hazel), water (aqua), Hamamelis virginiana (witch hazel) extract | | | | | 1.0 | 1.0 | |
| Extrapon Camomile GW (Symrise) | Glycerol, water (aqua), Chamomilla recutita (matricaria) flower extract | | 0.5 | | | | | |
| Extrapone Rosemary GW (Symrise) | Glycerol, water (aqua), Rosmarinus officinalis (rosemary) leaf extract | | 0.3 | | | | | |
| Frescolat ML crist. (Symrise) | Menthyl lactate | 0.5 | 0.5 | | 1.0 | 0.5 | 0.5 | 0.5 |
| Genapol LRO Liquid (Clariant) | Sodium laureth sulphate | | | | | | | 37.0 |
| Glycerol 85 P | Glycerol | 3.0 | 2.0 | 4.0 | | 4.7 | 2.0 | |
| Urea | Urea | | | 0.5 | | | 1.0 | |
| Hydrolite-5 | Pentylene glycol | 1.0 | 0.5 | | 3.0 | 3.0 | | |
| Hydroviton (Symrise) | Water, glycerol, sodium lactate, TEA lactate, serine, lactic acid, urea, sorbitol, sodium chloride, lauryl diethylene-diaminoglycine, lauryl aminopropylglycine, allantoin | 0.5 | 1.0 | | 1.0 | 1.0 | | 1.0 |
| Isodragol (Symrise) | Triisononanoin | | 2.0 | | | | | |
| Isopropyl palmitate (Symrise) | Isopropyl palmitate | 4.0 | | | | | | |
| Karion F (Merck) | Sorbitol | | | | | | 2.0 | |
| Keltrol RD (CP-Kelco) | Xanthan gum | 0.2 | 0.1 | | | | | |
| Keltrol T (Danby-Chemie) | Xanthan gum | | | | | 0.2 | | |
| Lanette 16 (Cognis) | Cetyl alcohol | 1.0 | | | | | | |
| Lanette O (Cognis) | Cetearyl alcohol | | 3.0 | | | 1.0 | | |
| Lara Care A-200 (Rahn) | Galactoarabinan | | | 0.3 | | | | |
| Magnesium sulphate (Merck) | Magnesium sulphate | | | | | | 0.7 | |
| Menthol (Symrise) | Menthol | | | 0.2 | | | | |
| Merquat 550 (Ondeo Nalco) | Polyquatemium-7 | | | | | | | 0.5 |
| Sodium benzoate | Sodium benzoate | | | | | | | 0.5 |
| Neo Heliopan 357 (Symrise) | Butyl methoxydibenzoylmethane | | | | | 1.0 | | |
| Neo Heliopan AP (Symrise) | Disodium phenyl dibenzimidazole tetrasulphonate | | | | | 4.6 | | |
| Neo Heliopan AV (Symrise) | Ethylhexyl methoxycinnamate | | | | | 3.0 | | |
| Neo Heliopan Hydro (Symrise) | Phenylbenzimidazole sulphonic acid | | | | | 6.7 | | |
| Neo Heliopan MBC (Symrise) | 4-Methylbenzylidene camphor | | | | | 1.5 | | |
| Neo Heliopan OS (Symrise) | Ethylhexyl salicylate | | | | | 5.0 | | |
| Neutral oil | Caprylic/capric triglyceride | 6.0 | | | 4.0 | 2.0 | | |
| Oxynex 2004 (Merck) | BHT | | | | | | 0.1 | |
| Paraffin oil 5 grade E (Parafluid) | Paraffinum liquidum | | | | 4.0 | | | |
| PCL Liquid 100 (Symrise) | Cetearyl ethylhexanoate | 3.0 | 5.0 | | 7.0 | | | |
| PCL Solid (Symrise) | Stearyl heptanoate, stearyl caprylate | | 2.0 | | | | | |
| PCL-Liquid (Symrise) | Cetearyl ethylhexanoate, isopropyl myristate | | | | | | 12.0 | |
| Pemulen TR-2 (Noveon) | Acrylates/C10-30 alkyl acrylate crosspolymer | | | 0.3 | 0.2 | | | |
| 1,2-Propylene glycol 99P GC | Propylene glycol | | 5.0 | | | | | |
| Sepigel 305 | Polyacrylamide, C13-14 isoparaffin, laureth-7 | | | | | | | |
| Sodium chloride | Sodium chloride | | | | | | | 1.0 |
| Sodium hydroxide (10 % solution) | Sodium hydroxide | | 0.3 | 0.6 | 0.4 | | | |
| Sunflower oil (Wagner) | Helianthus annuus (sunflower) seed oil | | | | | | 5.0 | |
| Sweet-almond oil (Wagner) | Prunus dulcis | | | | | | 5.0 | |
| Symdiol 68 (Symrise) | 1,2-Hexanediol, caprylyl glycol | 0.5 | | | | | | |
| Perfume oil (Symrise) | Fragrance | 0.3 | 0.3 | 0.3 | 0.2 | 0.4 | 0.4 | 0.5 |
| Tego Betaine L7 (Degussa) | Cocamidopropyl betaine | | | | | | | 6.0 |
| Tegosoft TN (Degussa) | C12-15 alkyl benzoate | | | 5.0 | | 5.0 | | |
| Triethanolamine 99 % | Triethanolamine | | | | | 0.5 | | |
| Retinyl palmitate in oil (DSM Nutritional Products) | Retinyl palmitate | | | | | | 0.2 | |
| Tocopherol acetate (DSM Nutritional Products) | Tocopheryl acetate | | | 0.5 | | 0.5 | 3.0 | |
| Water, demineralised | Water (aqua) | ad 100 | ad 100 | ad 100 | ad 100 | ad 100 | ad 100 | ad 100 |

Particularly preferred areas of application for the mixtures according to the invention are cosmetic products for treating itchy, dry skin, especially itchy, dry senile skin, sunscreen products (after-sun products), cosmetic products for treating stressed skin and a damaged skin barrier (due to detergents or soaps) and pharmaceutical compositions for treating diseases/skin damage associated with itching, such as, in particular, atopic dermatitis/neurodermatitis, psoriasis, urticaria, insect bites, allergic contact dermatitis, contact with allergenic plants, scabies, microbial infections, sunburn and other burns.

### Example S1: After-shave balm with anti-irritant, anti-itch and redness-reducing properties

| Ingredient | INCI-Name | A* wt.% | B wt.% | C wt.% | D wt.% |
|---|---|---|---|---|---|
| Water | Water (Aqua) | ad100 | ad100 | ad100 | ad100 |
| Ultrez-21 | Acrylates/C10-30 AlkylAcrylate Crosspolymer | 0.4 | 0.4 | 0.4 | 0.4 |
| Dragocide liquid | Phenoxyethanol, Methyl,-Ethyl,-Butyl,-Propyl,-Isobutylparaben | 0.8 | 0.8 | 0.8 | 0.8 |
| EDTA BD | Disodium EDTA | 0.1 | 0.1 | 0.1 | 0.1 |
| D-Panthenol | Panthenol | 1.0 | 1.0 | 1.0 | 1.0 |
| Glycerin 99% | Glycerin | 2.5 | 2.5 | 2.5 | 2.5 |
| Frescolat ML | Menthyl Lactate | - | 1.0 | 0.4 | 0.5 |
| L-Menthol | Menthol | - | - | 0.2 | - |
| Dihydroavenanthramide D (Formula 8) | Hydroxyphenyl Propamidobenzoic Acid | 0.05 | 0.025 | 0.025 | 0.025 |
| Abil 350 | Dimethicone | 3.0 | 3.0 | 3.0 | 3.0 |
| PCL Liquid 100 | Cetearyl Ethylhexanoate | 3.0 | 3.0 | 3.0 | 3.0 |
| Fragrance | Parfum | 0.5 | 0.5 | 0.5 | 0.5 |

| | | | | | |
|---|---|---|---|---|---|
| A*: reference, see WO 2004/047833; not according to the present invention | | | | | |

Each after-shave balm was used by 10 male testers and applied to the facial skin directly after shaving. Balms B, C, and D according to the invention resulted in less ichting and reddening and were found less irritant when compared to formulation A*.

### Example S2: Shaving foam with anti-irritant, anti-itch and redness-reducing properties

| Ingredient | INCI-Name | A* wt.% | B wt.% | C wt.% | D wt.% |
|---|---|---|---|---|---|
| Water | Water (Aqua) | ad100 | ad100 | ad100 | ad100 |
| Triethanolamine 99% | Triethanolamine | 4.0 | 4.0 | 4.0 | 4.0 |
| Frescolat MGA | Menthone Glycerin Acetal | - | - | 0.4 | 0.25 |
| L-Menthol | Menthol | - | 0.25 | 0.1 | 0.25 |
| Dihydroavenanthramide D (Formula 8) | Hydroxyphenyl Propamidobenzoic Acid | 0.05 | 0.04 | 0.025 | 0.025 |
| Edenor L2SM | Stearic Acid, Palmitic Acid | 5.3 | 5.3 | 5.3 | 5.3 |
| Brij 35 | Laureth-23 | 3.0 | 3.0 | 3.0 | 3.0 |
| Lanette 18 | Stearyl Alcohol | 0.5 | 0.5 | 0.5 | 0.5 |
| Dragocide liquid | Phenoxyethanol, Methyl,-Ethyl,-Butyl,-Propyl,-Isobutylparaben | 0.8 | 0.8 | 0.8 | 0.8 |
| | | | | | |
| | | | | | |
| Fragrance | Parfum | 0.5 | 0.5 | 0.5 | 0.5 |
| Texapon NSO | Sodium Laureth Sulfate | 3.0 | 3.0 | 3.0 | 3.0 |
| Propane Butane 4,2 bar | Propane-Butane | 4.0 | 4.0 | 4.0 | 4.0 |

| | | | | | |
|---|---|---|---|---|---|
| A*: reference, see WO 2004/047833; not according to the present invention The shaving foam had a pH-value of 8,5. | | | | | |

Each shaving foam was used by 10 male testers and the shaved skin assessed after shaving. Foams B, C, and D according to the invention resulted in less ichting and reddening and were found less irritant when compared to formulation A*. Overall less razor burn was experienced.

### Example N1: Atomizing nasal pump spray with anti-irritant, anti-itch and redness-reducing properties

| Ingredient | INCI-Name | A* wt.% | B wt.% | C wt.% | D wt.% |
|---|---|---|---|---|---|
| Water | Water (Aqua) | ad100 | ad100 | ad100 | ad100 |
| Frescolat MGA (Symrise) | Menthone Glycerin Acetal | - | - | 0.10 | 0.15 |
| L-Menthol | Menthol | - | 0.10 | 0.05 | - |
| Dihydroavenanthramide D (Formula 8) | Hydroxyphenyl Propamidobenzoic Acid | 0.05 | 0.03 | 0.025 | 0.05 |
| Polysorbate 20 | Polysorbate 20 | 0.50 | 0.50 | 0.50 | 0.50 |
| Benzalkonium chloride | Benzalkonium chloride | 0.01 | 0.01 | 0.01 | 0.01 |
| Sodium chloride | Sodium chloride | 0.10 | 0.10 | 0.10 | 0.10 |
| EDTA BD | Disodium EDTA | 0.01 | 0.01 | 0.01 | 0.01 |

| | | | | | |
|---|---|---|---|---|---|
| A*: reference, see WO 2004/047833; not according to the present invention | | | | | |

Each nasal spray was used by 10 testers (5 female and 5 male) and the nasal skin assessed 5, 15 and 30 minutes after use of the respective nasal spray. Pump sprays B, C, and D according to the invention resulted in less ichting and reddening and were found less irritant when compared to formulation A*.

### Example N2: Atomizing nasal pump spray with anti-irritant, anti-itch and redness-reducing properties

| Ingredient | INCI-Name | A* wt.% | B wt.% | C wt.% | D wt.% |
|---|---|---|---|---|---|
| Water | Water (Aqua) | ad100 | ad100 | ad100 | ad100 |
| Frescolat MGA (Symrise) | Menthone Glycerin Acetal | - | - | 0.10 | 0.15 |
| L-Menthol | Menthol | - | 0.10 | 0.05 | - |
| Avenanthramide D (Formula 10) | | 0.05 | 0.03 | 0.025 | 0.05 |
| Glycerin | Glycerin | 2.00 | 2.00 | 2.00 | 2.00 |
| Polysorbate 80 | Polysorbate 80 | 0.50 | 0.50 | 0.50 | 0.50 |
| Phenylethyl alcohol | 2-Phenylethanol | 0.20 | 0.20 | 0.20 | 0.20 |
| Benzalkonium chloride | Benzalkonium chloride | 0.02 | 0.02 | 0.02 | 0.02 |
| Fluticasone propionate^ | Sodium chloride | 0.10 | 0.10 | 0.10 | 0.10 |
| Citric acid | Citric acid | 0.01 | 0.01 | 0.01 | 0.01 |

| | | | | | |
|---|---|---|---|---|---|
| A*: reference, see WO 2004/047833; not according to the present invention ^: S-(fluoromethyl)6a,9-difluoro-11ß-17-dihydroxy-16a-methyl-3-oxoandrosta-1,4-diene-17ß-carbothioate, 17-propionate | | | | | |

Each nasal spray was used by 10 testers (5 female and 5 male) and the nasal skin assessed 5, 15 and 30 minutes after use of the respective nasal spray. Pump sprays B, C, and D according to the invention resulted in less ichting and reddening and were found less irritant when compared to formulation A*.

### Example W1: Wet wipes with anti-irritant, anti-itch and redness-reducing properties

An emulsion consisting of the following ingredients was produced and impregnated on a non-woven substrate to produce wet wipes (e.g. as described in WO 02/19984).

| Ingredient | A* wt.% | B wt.% | C wt.% | D wt.% |
|---|---|---|---|---|
| Water | ad100 | ad100 | ad100 | ad100 |
| Frescolat ML (Menthyl lactate) | - | - | 0.10 | 0.15 |
| L-Menthol | - | 0.10 | 0.05 | - |
| Dihydroavenanthramide D (Formula 8) | 0.05 | 0.03 | 0.025 | 0.05 |
| C10-C30 Alkyl acrylate cross polymer | 0.125 | 0.125 | 0.125 | 0.125 |
| Salicylic acid | 0.50 | - | 0.20 | - |
| Poloxamer 124 | 0.03 | 0.03 | 0.03 | 0.03 |
| Polysorbate 20 | 0.05 | 0.05 | 0.05 | 0.05 |
| Cyclomethicone | 0.20 | 0.20 | 0.20 | 0.20 |
| Dimethicone | 0.10 | 0.10 | 0.10 | 0.10 |
| C12-C15 Alkyl benzoate | 0.50 | 0.50 | 0.50 | 0.50 |
| Fragrance | 0.05 | 0.05 | 0.04 | 0.06 |
| Butylene glycol | 1.00 | 1.00 | 1.00 | 1.00 |
| Propylene glycol | 2.00 | 2.00 | 2.00 | 2.00 |
| PHENONIP | 0.20 | - | 0.30 | - |
| Sodium hydroxide | 0.425 | 0.425 | 0.425 | 0.425 |
| Allantoin | 0.20 | - | 0.10 | 0.10 |
| Aloe Barbadensis | - | 0.20 | 0.10 | 0.15 |
| Vitamin E - acetate (Tocopheryl Acetate) | - | 0.10 | 0.05 | - |
| lodopropynyl Butylcarbamate | 0.01 | 0.01 | - | - |
| 1,2-Hexanediol, 1,2-Octanediol (1:1) (m/m) | - | 0.50 | - | 0.90 |
| Sodium benzoate | - | - | 0.15 | - |
| Alpha-Bisabolol | 0.06 | 0.05 | 0.10 | - |

| | | | | |
|---|---|---|---|---|
| A*: reference, see WO 2004/047833; not according to the present invention PHENONIP is a commercially available mixture of phenoxyethanol, methylparaben, ethylparaben, propylparaben and butylparaben. | | | | |

The wet wipes thus produced were used by 10 panellists (5 female and 5 male) on the nose and the naso-labial skin. The skin was assessed 5, 10 and 15 minutes after use of the respective wet wipe.

## Claims

1. Mixture comprising or consisting of:
(a) a compound of Formula 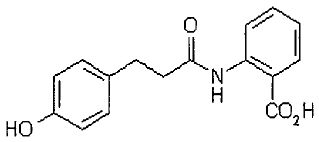 also representing the corresponding cosmetically or pharmaceutically acceptable salts and solvates;
and
(b) one or more cooling agents selected from the group consisting of l-menthol, menthone glycerol acetal and menthyl lactate (preferably I-menthyl lactate, especially I-menthyl I-lactate).

2. Mixture according to claim 1 wherein the amount of compound of Formula 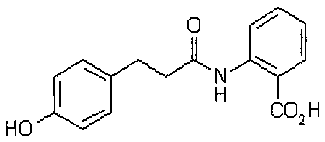 and/or the amount of cooling agent(s), each taken by itself, has no action in the alleviation of itching or the reduction of skin reddening, but the total amount of compound of Formula 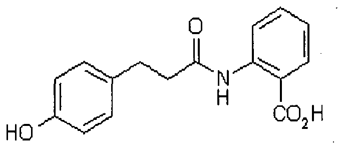 and cooling agent(s) does have such an action.

3. Mixture according to one of the preceding claims as a cosmetic composition for use in the treatment or prevention of itching (pruritus) and/or the reduction of skin reddening.

4. Cosmetic method of reducing skin reddening, comprising the following step: application to the skin of a mixture according to one of claims 1 to 3 in an amount that reduces skin reddening.

5. Cosmetic or pharmaceutical product comprising a mixture according to any of claims 1 to 3, preferably in an amount that alleviates itching and/or reduces skin reddening, selected from the group consisting of soap, synthetic detergent, liquid washing, shower and bath preparation, solution, dispersion, suspension, cream, lotion, milk, W/O-, O/W- or multiple emulsion, PIT emulsion, emulsion foam, micro-emulsion, nano-emulsion, Pickering emulsion, ointment, paste, gel (including hydrogel, hydrodispersion gel, oleogel), oil, toner, balsam, serum, powder, eau de toilette, toilette, eau de cologne, perfume, wax, stick, roll-on, (pump) spray, nasal spray, nasal drops, aerosol (foaming, non-foaming or post-foaming), (O/W) day cream, (O/W) skin lotion, (W/O) night cream, body spray, suncreen lotion, after-sun product (balm, lotion, milk), foot care product (including keratolytics, deodorants), shaving foam, aftershave (balm, lotion), depilatory product, hair care product [e.g. shampoo (including 2-in-1 shampoo), conditioner, hair tonic, hair water, hair rinse, hair cream, pomade, perm and setting lotion, hair smoothing product (detangling product, relaxer), hair strengthener, styling aid (e.g. gel or wax), blonding product, hair dye (e.g. temporary hair dyes, colour rinses, semi-permanent and permanent hair dyes)], nail care products (e.g. nail polish and nail polish remover), deodorant, antiperspirant, mouthwash, (wet) wipe, suppository, ophthalmic preparation (e.g. drops, ointment), makeup, makeup remover, decorative cosmetics (e.g. powder, eyeshadows, kohl pencil, lipstick).

6. Shaving or after-shave product comprising a mixture according to any of claims 1 to 3, preferably in an amount that alleviates itching and/or reduces skin reddening.

7. Product according to claim 6, wherein the shaving or after-shave product is selected from the group consisting of shaving foam, shaving gel, shaving soap, shaving lather, after-shave balm, after-shave lotion, after-shave splash, and after-shave.

8. Cosmetic or pharmaceutical product comprising a mixture according to any of claims 1 to 3, preferably in an amount that alleviates itching and/or reduces skin reddening.

9. Product according to claim 8, wherein the product is selected from the group consisting of nasal spray, nasal drops, nasal ointment, wipe, wet wipe, and ophthalmic preparation.

## Patentansprüche

1. Mischung,umfassend oder bestehend aus :
(a) eine Komponente der Formel 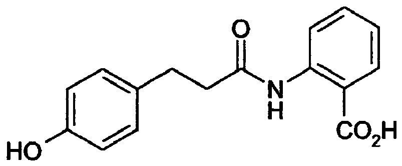 auch bedeutend für die korrespondierenden pharmazeutisch akzeptablen Salze und Solvate; und
(b) eine oder mehr Kühlwirkstoffe ausgewählt aus der Gruppe bestehend aus I-Menthol, Menthone Glycerolacetal und Menthyl Lactat (vorzugsweise I-Menthyl Lactat, insbesondere l-Menthyl I-Lactat).

2. Mischung nach Anspruch 1, worin die Menge der Komponente der Formel 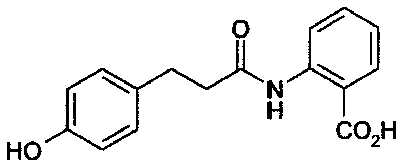 und/oder die Menge von Kühlwirkstoff(en), jede(s) für sich genommen dabei keine Aktivität in der Linderung von Juckreiz oder der Reduktion von Hautrötungen aufzeigt, jedoch die Gesamtmenge der Komponente der Formel 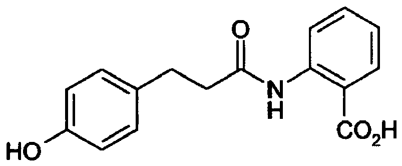 und Kühlwirkstoffe(n)zusammen, eine solche Aktivität hat.

3. Mischung nach einem der vorhergehenden Ansprüchen als eine kosmetische Zusammensetzung zur Verwendung bei der Behandlung oder Prävention von Juckreiz (Pruritus)und/oder Reduktion von Hautrötungen.

4. Kosmetisches Verfahren zur Reduktion von Hautrötung, umfassend die folgenden Schritte : Anwendung einer Mischung nach einem der Ansprüche 1 bis 3 auf die Haut in einer Menge, die die Hautrötungen reduziert.

5. Kosmetische oder pharmazeutische Produkte umfassend eine Mischung nach einem der Ansprüche 1 bis 3, vorzugsweise in einer Menge die Juckreiz lindert und/oder Hautrötung reduziert, ausgewählt aus der Gruppe bestehend aus Seife, synthetische Reinigungsmittel, flüssige Spülung, Dusch und Badezusammensetzung, Lösung, Dispersion, Suspension, Creme, Lotion, Milch, W/O-, O/W- oder multiple Emulsion, PIT Emulsion, Schaumemulsion, Mikro-Emulsion, Nano-Emulsion, Pickering Emulsion, Salbe, Paste, Gel (einschließlich Hydrogel, Hydrodispersion Gel, Oleogel) Öl, Wässerchen, Balsam, Serum, Pulver, Eau de Toilette, Eau de Cologne, Parfüm, Wachs, Stick, Deoroller, (Pump) Spray, Nasalspray, Nasaltropfen, Aerosol (schäumend, nicht-schäumend oder post-schäumend)(o/w) Tagescreme, (O/W) Hautlotion, (W/O) Nachtcreme, Körperspray, Sonnencreme-Lotion, After-Sun Produkte (Balsam, Lotion, Milch), Fusspflegeprodukte (einschließlich Keratolyten, Deodorantien) Rasierschaum, Aftershave (Balsam, Lotion), Enthaarungsprodukte, Haarpflegeprodukte [z.B. Shampoo (einschließlich 2-in-1 Shampoo)],Konditioner, Haartonikum, Haarwasser, Haarspülung, Haarcreme, Pomade, Dauerwelle und Festiger Lotion, Haarglättungsprodukte (Entwirrungsprodukt, Relaxer) Haarfestiger, Styling-Spülungen (z.B. Gel oder Wachs), Blondiermittelprodukt, Haarfärbemittel (z.B. temporäre Haarfärbemittel, Farbspülungen, semi-permament und permanente Haarfärbemittel), Nagelpflegeprodukte (z.B. Nagellack und Nagellackentferner), Deodorant, Antitranspirant, Mundwasser, (nass) Tücher, Zäpfchen, ophthalmische Zusammensetzung (z.B. Tropfen, Salbe), Make-up, Make-up Entferner, dekorative Kosmetik (z.B. Pulver, Lidschatten, Kajalstift, Lippenstift).

6. Rasur oder After-Shave-Produkt umfassend eine Mischung nach einem der Ansprüche 1 bis 3, vorzugsweise in einer Menge die Juckreiz lindert und/oder Hautrötungen reduziert.

7. Produkt nach Anspruch 6, wobei das Rasur oder After-Shave-Produkt ausgewählt ist aus der Gruppe bestehend aus Rasierschaum, Rasiergel, Rasierseife, Rasierseifenschaum, After-Shave Balsam, After-Shave Lotion, After-Shave Spritzwasser, und After-Shave.

8. Kosmetisches oder pharmazeutisches Produkt umfassend eine Mischung nach einem der Ansprüche 1 bis 3, vorzugsweise in einer Menge die Juckreiz lindert und/oder Hautrötungen reduziert.

9. Produkt nach Anspruch 8, wobei das Produkt ausgewählt ist aus der Gruppe bestehend aus Nasalspray, Nasaltropfen, Nasalsalbe, Wischtuch, Feuchttuch und ophthlamische Zusammensetzungen.

## Revendications

1. Mélange comprenant, ou étant constitué de :
(a) un composé de Formule 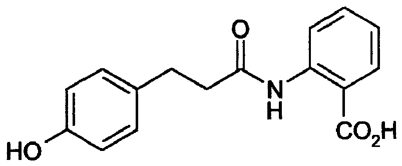 représentant également les sels et solvats correspondants cosmétiquement ou pharmaceutiquement acceptables ;
et
(b) un ou plusieurs agents de refroidissement choisis dans le groupe constitué par le l-menthol, l'acétal de glycérol et de menthone et le lactate de menthyle (préférablement le lactate de I-menthyle, notamment le I-lactate de l-menthyle).

2. Mélange selon la revendication 1, dans lequel la quantité de composé de Formule 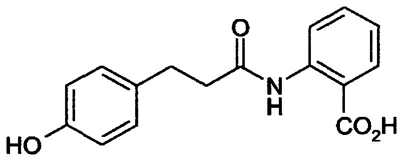 et/ou la quantité du ou des agents de refroidissement, chacun pris seul, n'a aucune action dans le soulagement des démangeaisons ou la réduction du rougissement de la peau, mais la quantité totale du composé de Formule 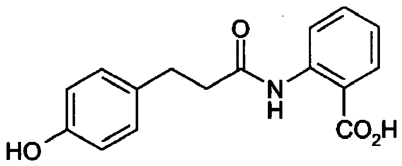 et du ou des agents de refroidissement, possède bien une telle action.

3. Mélange selon l'une des revendications précédentes, comme composition cosmétique pour une utilisation dans le traitement ou la prévention des démangeaisons (prurit) et/ou la réduction du rougissement de la peau.

4. Méthode cosmétique de réduction du rougissement de la peau, comprenant l'étape suivante : l'application à la peau d'un mélange selon l'une des revendications 1 à 3 selon une quantité qui réduit le rougissement de la peau.

5. Produit cosmétique ou pharmaceutique comprenant un mélange selon l'une quelconque des revendications 1 à 3, préférablement selon une quantité qui soulage les démangeaisons et/ou réduit le rougissement de la peau, choisi dans le groupe constitué par un savon, un détergent synthétique, un nettoyant liquide, une préparation pour la douche et le bain, une solution, une dispersion, une suspension, une crème, une lotion, un lait, une émulsion E/H, H/E ou multiple, une émulsion PIT, une mousse émulsionnée, une microémulsion, une nano-émulsion, une émulsion de Pickering, un onguent, une pâte, un gel (y compris un hydrogel, un gel de type hydrodispersion, un oléogel), une huile, un tonique, un baume, un sérum, une poudre, une eau de toilette, une eau de Cologne, un parfum, une cire, un bâtonnet, un roll-on, une pulvérisation (à pompe), une pulvérisation nasale, des gouttes nasales, un aérosol (moussant, non moussant ou post-moussant), une crème de jour (H/E), une lotion pour la peau (H/E), une crème de nuit (E/H), une pulvérisation pour le corps, une lotion d'écran solaire, un produit après-soleil (baume, lotion, lait), un produit de soin des pieds (y compris les produits kératolytiques, les déodorants), une mousse à raser, un aftershave (baume, lotion), un produit dépilatoire, un produit de soin capillaire [par exemple un shampooing (y compris un shampooing 2 en 1), un après-shampooing, un tonique capillaire, une eau capillaire, un rinçage capillaire, une crème capillaire, une pommade, une lotion de permanente et de mise en plis, un produit de lissage capillaire (produit démêlant, défrisant), un renforçateur capillaire, un auxiliaire de coiffage (par exemple un gel ou une cire), un produit éclaircissant, un colorant capillaire (par exemple des colorants capillaires temporaires, des rinçages colorants, des colorants capillaires semi-permanents et permanents)], des produits de soin des ongles (par exemple des vernis à ongles et des dissolvants à ongles), un déodorant, un anti-transpirant, un bain de bouche, une lingette (humide), un suppositoire, une préparation ophtalmique (par exemple des gouttes, un onguent), un produit de maquillage, un démaquillant, des produits cosmétiques décoratifs (par exemple une poudre, un fard à paupières, un crayon khôl, un bâton de rouge à lèvres).

6. Produit de rasage ou d'après-rasage comprenant un mélange selon l'une quelconque des revendications 1 à 3, préférablement selon une quantité qui soulage les démangeaisons et/ou réduit le rougissement de la peau.

7. Produit selon la revendication 6, **caractérisé en ce que** le produit de rasage ou d'après-rasage est choisi dans le groupe constitué par une mousse à raser, un gel de rasage, un savon à barbe, une mousse pour rasage, un baume après-rasage, une lotion après-rasage, une eau de Cologne après-rasage, et un after-shave.

8. Produit cosmétique ou pharmaceutique comprenant un mélange selon l'une quelconque des revendications 1 à 3, préférablement selon une quantité qui soulage les démangeaisons et/ou réduit le rougissement de la peau.

9. Produit selon la revendication 8, **caractérisé en ce que** le produit est choisi dans le groupe constitué par une pulvérisation nasale, des gouttes nasales, un onguent nasal, une lingette, une lingette humide, et une préparation ophtalmique.
